(19) Europäisches Patentamt
European Patent Office
Office européen des brevets

(11) Numéro de publication: **0 406 112 B1**

# (12) FASCICULE DE BREVET EUROPEEN

(45) Date de publication de fascicule du brevet: **28.12.94** (51) Int. Cl.5: **C07D 205/04**

(21) Numéro de dépôt: **90401860.3**

(22) Date de dépôt: **28.06.90**

Le dossier contient des informations techniques présentées postérieurement au dépôt de la demande et ne figurant pas dans le présent fascicule.

## (54) 1-Benzhydrylazétidines, leur préparation et leur application comme intermédiaires pour la préparation de composés à activité antimicrobienne.

(30) Priorité: **29.06.89 FR 8908696**

(43) Date de publication de la demande: **02.01.91 Bulletin 91/01**

(45) Mention de la délivrance du brevet: **28.12.94 Bulletin 94/52**

(84) Etats contractants désignés: **AT BE CH DE DK FR GB GR IT LI LU NL SE**

(56) Documents cités:
**EP-A- 0 153 163**
**EP-A- 0 155 870**
**DE-A- 3 627 246**
**FR-A- 2 150 816**

**J. Heterocycl. Chem., vol. 6, no. 3, pp.273-277, 1969**

(73) Titulaire: **LABORATORIOS DEL DR. ESTEVE, S.A.**
**Av. Mare de Deu de Montserrat, 221**
**E-08026 Barcelona (ES)**

(72) Inventeur: **Frigola-Constansa, Jordi**
**Av. Diagonal 299 at. 1a**
**E-08013 Barcelone (ES)**
Inventeur: **Colombo-Pinol, Augusto**
**Av. Chile, 36, 40 1a**
**E-08032 Barcelona (ES)**
Inventeur: **Pares-Corominas, Juan**
**Padilla 349, 30 3a**
**E-08025 Barcelona (ES)**

(74) Mandataire: **Ahner, Francis et al**
**CABINET REGIMBEAU**
**26, avenue Kléber**
**F-75116 Paris (FR)**

EP 0 406 112 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux intermédiaires chimiques répondant à la formule générale I, leur procédé de préparation, ainsi que leur application pour la préparation de substances biologiquement actives.

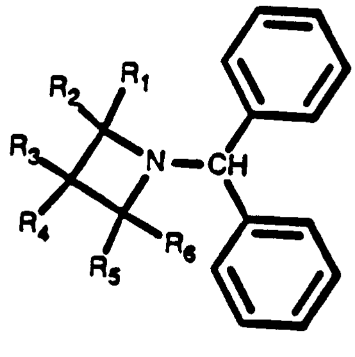

(I)

Les composés de formule générale II

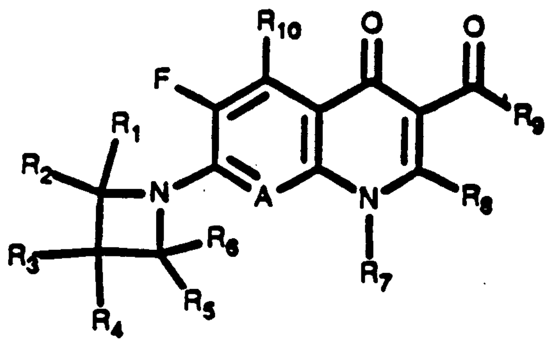

(II)

ont été décrits dans le brevet européen EP-A-0 324 298 et les brevets français FR-A-2 644 455 et FR-A-2 645 862. Quelques-uns de ces composés sont en train d'être développés à cause de leurs excellentes activités antimicrobiennes.

Il est important d'obtenir des intermédiaires simples et efficients et des routes synthétiques pour les composés de formule général II qui contiennent le groupement 1-azétidinyl (III)

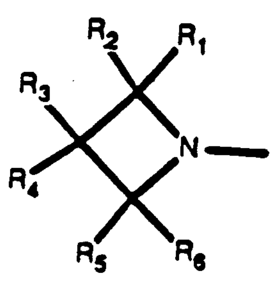

(III)

On connaît dans la littérature scientifique quelques dérivés de formule générale (I) dans laquelle $R_3$ représente un radical amino, alkylamino, dialkylamino, aminométhyl ou alkylaminométhyl, mais il n'existe aucun exemple de composés de formule générale (I) dans laquelle $R_3$ a la signification mentionnée et l'un au moins des autres substituants représente simultanément un radical alkyl inférieur. Les composés de formule générale (I) amino substitués décrits dans la littérature scientifique jusqu'à présent se trouvent dans Chemical Abstracts, 105(15) : 133737d, 104(23) : 207074g, 104(5) : 34013n, 101(9) : 72740t, 78(21) : 135969d, 83(1) : 9760u, 90(11) : 80717k et 78(3) :15930n, et la demande de brevet FR-A-2 150 816.

Nous avons maintenant découvert, selon la présente invention, que les composés de formule générale (I), dans laquelle $R_3$ représente un radical amino, un radical alkylamino, un radical dialkylamino, un radical

cycloalkylamino, un radical acylamino, un radical alkylacylamino, un radical aminométhyle, un radical alkylaminométhyle, un radical acylaminométhyle ou un radical alkylacylaminométhyle, dans lesquels chaque fragment acyle peut être substitué par un ou plusieurs atomes d'halogène, en particulier de fluor ;

$R_1$, $R_2$, $R_4$, $R_5$ et $R_6$ représentent un atome d'hydrogène ou un radical alkyl inférieur, avec la condition que au moins un d'eux représente un radical alkyl inférieur, sont des composés nouveaux et utiles comme intermédiaires pour la préparation de composés thérapeutiquement actifs, tels que les quinolones, naphtyridines, pyridobenzoxazines, thiazetoquinolones, benzoquinolizines, benzothiazoloquinolones, pyridobenzothiazines, benzoxazoloquinolones, époxyméthanothiazoloquinolones et isothiazoloquinolines de formule générale (II).

Les nouvelles azétidines de formule générale (I) peuvent avoir, selon le nombre, la nature et la position relative des substituants, jusqu'à trois centres chiraux, chacun d'eux avec une configuration "R" ou "S".

Les composés de formule générale (I) dans laquelle $R_1$, $R_2$, $R_3$, $R_4$, $R_5$ et $R_6$ ont la signification mentionnée ci-dessus, sont des produits obtenus par substitution de $R_3$ lorsqu'il représente un bon "groupe partant" par un nucléophile adéquat, sans toutefois nécessiter une étape intermédiaire de substitution par un groupe phtalimido, telle que décrite dans la demande de brevet EP-A-0 155 870. Cette substitution est très avantageuse, en ce qui concerne les rendements, à cause de la présence du groupement benzidryl comme groupement protecteur de l'azétidine en position 1. De surcroît, le groupement benzidryl est également très avantageux en ce qui concerne les rendements pour la déprotection qui conduit aux azétidines de formule générale (IV), nécessaires pour préparer les composés de formule générale (II) tel qu'il a été décrit dans les brevets EP-A-324298 ; EP-A-0 388 298 et EP-A-0 394 120 (état de la technique visé à l'article 54(3) CBE).

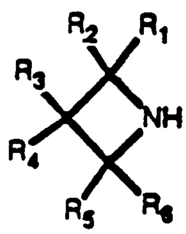

(IV)

Un autre avantage additionnel dans l'utilisation du radical benzidryl comme groupement protecteur dans les composés de formule générale (I), se trouve dans les cas où il existe des carbones chiraux, puisque lorsque l'on effectue les réactions de substitution mentionnées antérieurement, on observe une rétention de configuration qui évite ainsi la formation de mélanges de stéréoisomères. En outre, les composés de formule générale (I) sont très appropriés pour effectuer la résolution des mélanges racémiques moyennant l'utilisation d'enzymes ou d'acides organiques énantiomériquement purs.

La présente invention se rapporte aussi à la préparation des composés de formule générale (I). Les nouveaux dérivés objet de l'invention peuvent être préparés selon le schéma 1, qui conduit par tous les chemins aux intermédiaires de formule générale (XI) qui, par des réactions successives donnent lieu aux composés de formule générale (I). Dans les composés (V) à (XIII) du schéma 1, les radicaux $R_1$, $R_2$, $R_4$, $R_5$ et $R_6$ ont la signification mentionnée ci-dessus, X représente un atome de chlore, un atome de brome, un radical hydroxyle, un radical acétyloxy, un radical méthylsulfonyloxy, un radical p-toluènesulfonyloxy, un radical p-nitrobenzoyloxy, un radical 2-naphtalénesulfonyloxy, un radical tert-butyldiphénylsilyloxy ou un radical tert-butyldiméthylsilyloxy, Y représente un atome de chlore ou un atome de brome, et Z représente un radical méthylsulfonyloxy, un radical p-toluénesulfonyloxy ou un radical cyano.

Selon un de ses aspects, la présente invention a pour objet la préparation des composés de formule générale (I) selon le schéma 1 :

# Schéma 1

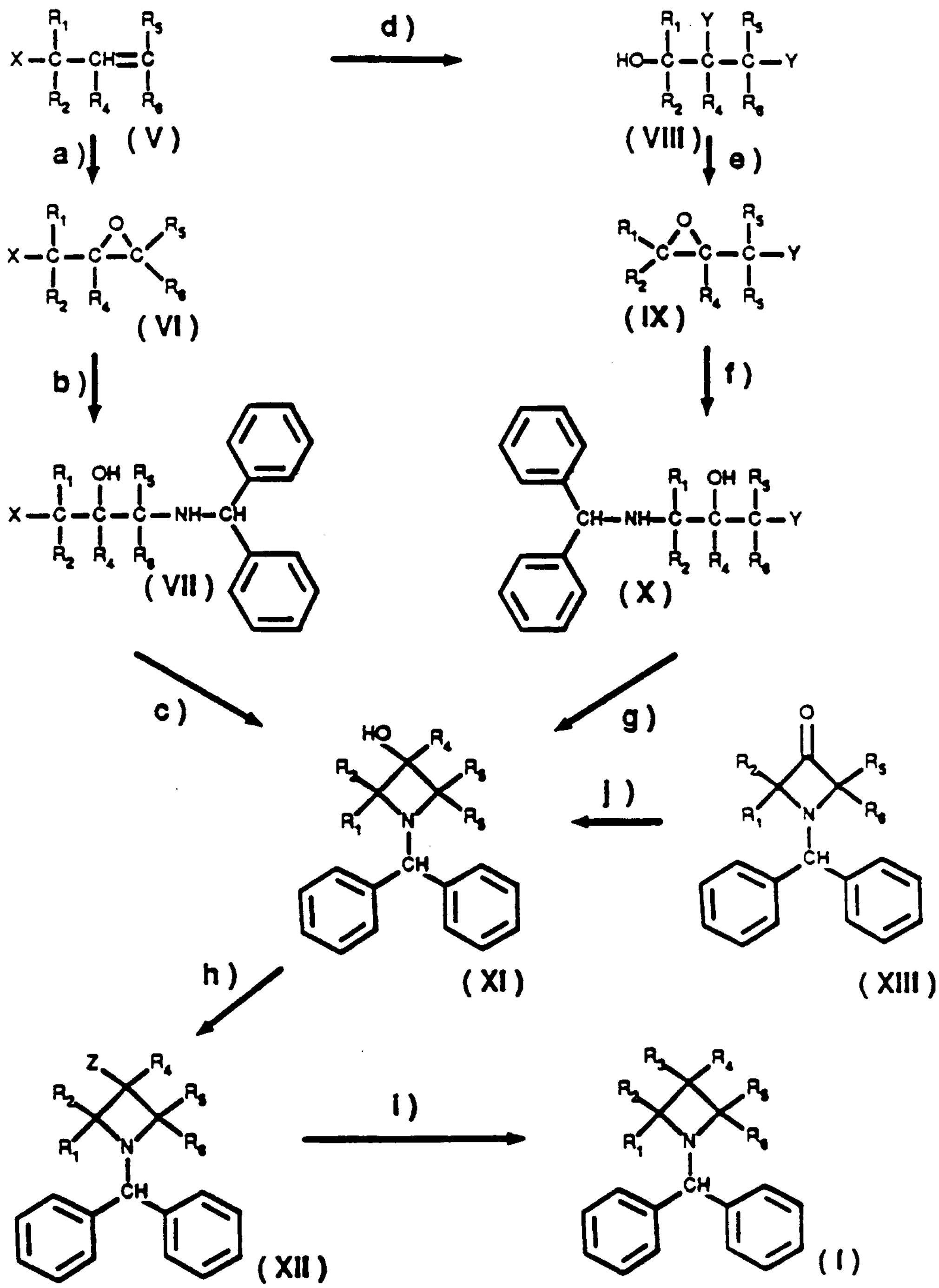


Synthèse des époxydes (VI) ou (IX)

a) Par réaction d'un composé de formule générale (V) avec un agent oxydant tel que l'acide peracétique, l'acide m-chlorperbenzoïque, la N-bromosuccinimide ou le tert-butylhydroperoxyde, on obtient l'époxyde

de formule générale (IV).

Lorsque l'on utilise le N-bromosuccinimide comme agent oxydant, on effectue la réaction dans l'eau suivant une méthode préalablement décrite (A.H. Yavronian, R.A. Sanchez, J.K. Pollard et E.K. Metzner, Synthesis, 1981, 791). Dans le cas où l'on effectue une époxydation assymétrique, le réactif préféré est le tert-butylhydroperoxyde en présence d'isopropoxyde de titanium et de tartrate de diéthyle, et pour la résolution cinétique, on peut utiliser le tartrate d'isopropyle, le tartrate de dicyclohexyle ou le tartrate de dicyclododécyle tel qu'il a été décrit préalablement (Y. Gao, M. Hanson, J.M. Klunder, S.Y. Ko, H. Masamune et K.B. Sharpless, J. Am. chem. Soc., 1987, 109, 5765).

d,e) Par addition de brome ou de chlore aux composés de formule générale (V) dans lesquels X représente OH, et traitement ultérieur avec une base telle que l'hydroxyde de potassium, on obtient les époxydes de formule générale (IX), tel qu'il a été décrit antérieurement pour quelques composés (C.F. Hiskey, H.L. Slates et N.L. Wendler, J. Org. Chem., 1956, 21, 429 ; R.H. Higgins et N.H. Cromwell, J. Heterocyclic Chem., 1971, 8 1059). La réaction s'effectue sans procéder à l'isolement des composés intermédiaires de formule générale (VIII).

Synthèse des azétidinols (XI)

b,c) ou f,g) Les composés de formule générale (XI) se synthétisent à partir des composés de formule générale (VI) ou (IX) par réaction avec de la diphénylméthylamine sans isoler les produits intermédiaires (VII) ou (X) respectivement.

On effectue la réaction au sein d'un solvant polaire tel que le méthanol ou l'éthanol, pendant un temps compris entre deux jours et dix jours. La température appropriée pour effectuer l'ouverture de l'époxyde oscille entre 0 °C et 30 °C et la température appropriée pour la cyclation oscille entre 20 °C et la température d'ébullition du solvant utilisé.

j) Les cétones de formule générale (XIII) par traitement avec de l'alkylithium ($R_4Li$) ou des halogénures d'alkylmagnésium ($R_4MgX$) donnent lieu aux correspondants 3-alkyl-3-azétidinols de formule générale (XI) tel qu'il a été préalablement décrit (S.S. Chatterjee et A. Shoeb, Synthesis, 1973, 153).

Synthèse des azétidines de formule générale (XII)

h1) Les composés de formule générale (XII) dans lesquels Z représente un radical méthylsulfonyloxy ou un radical p-toluènesulfonyloxy, se synthétisent par réaction des composés de formule générale (XI) avec du chlorure de l'acide méthansulfonique ou du chlorure de l'acide p-toluénesulfonique, respectivement. La réaction s'effectue de préférence dans des solvants chlorés, tels que le chlorure de méthylène ou le chloroforme, d'amines tertiaires, telles que la pyridine ou la triéthylamine, ou bien des mélanges de ces solvants. La réaction s'effectue à des températures comprises entre -30 °C et 40 °C pendant des temps compris entre 1 heure et 24 heures.

h2) Les composés de formule générale (XII) dans lesquels Z représente un radical cyano, se synthétisent par réaction des composés de formule générale (XII) dans lesquels Z représente un radical méthylsulfonyloxy ou un radical p-toluènesulfonyloxy avec du cyanure de sodium. La réaction s'effectue en utilisant un solvant approprié tel que la diméthylformamide ou le diméthylsulfoxyde. On maintient sous agitation pendant un temps compris entre 3 et 8 heures, à une température qui oscille entre 50 °C et 100 °C.

Synthèse des azétidines de formule générale (I)

i1) Les composés de formule générale (I) dans lesquels $R_3$ représente un radical amino, un radical alkylamino, un radical dialkylamino ou un radical cycloalkylamino, s'obtiennent par réaction d'un composé de formule générale (XII) dans lequel Z représente un radical méthylsulfonyloxy ou p-toluénesulfonyloxy avec de l'ammoniaque, une alkylamine, une dialkylamine ou une cycloalkylamine respectivement. La réaction s'effectue dans un solvant approprié, à des températures comprises entre 40 °C et 120 °C pendant des temps compris entre 1 heure et 48 heures, tant sous pression atmosphérique que dans un récipient fermé. Les solvants appropriés sont l'eau, les solvants dipolaires aprotiques tels que la diméthylformamide ou le diméthylsulfoxyde. Les alcools tels que l'éthanol ou l'isopropanol, les éthers tels que le tétrahydrofuranne ou le dioxanne, ou bien un mélange de deux des solvants indiqués.

i2) Les composés de formule générale (I) dans lesquels $R_3$ représente un radical aminométhyl s'obtiennent par réduction d'un composé de formule générale (XII) dans lequel Z représente un radical

cyano. Cette réduction s'effectue de préférence avec le concours d'un hydrure métallique tel que l'hydrure d'aluminium et de lithium dans un éther tel que le diéthyléther ou le tétrahydrofuranne. La température se maintient entre 25 et 40 ° C pendant l'addition du nitrile et postérieurement on agite pendant un temps compris entre 12 et 24 heures à température ambiante (25 ° C).

i3) Les composés de formule générale (I) dans lesquels $R_3$ représente un radical acylamino, un radical alkylacylamino ou un radical acylaminométhyl, s'obtiennent par réaction des composés de formule générale (I) dans lesquels $R_3$ représente un radical amino, un radical alkylamino ou un radical aminométhyl respectivement, avec un chlorure d'acide carboxylique ou avec un anhydride d'acide carboxylique. Cette réaction s'effectue de préférence dans un hydrocarbure aromatique tel que le benzène ou le toluène, d'un composé chloré tel que le chlorure de méthylène ou le chloroforme, ou bien des mélanges de ces solvants. De plus, la présence d'une base inorganique telle que le carbonate de sodium ou organique telle que la pyridine ou la triéthylamine, est souhaitable. Les températures appropriées oscillent entre 0 ° C et 30 ° C pendant un temps compris entre 1 heure et 4 heures.

i4) Les composés de formule générale (I) dans lesquels $R_3$ représente un radical alkylacylaminométhyl s'obtiennent par alkylation des composés de formule générale (I) dans lesquels $R_3$ représente un radical acylaminométhyl, en utilisant comme agent alkylant un halogénure d'alkyle. La réaction s'effectue suivant un procédé analogue à celui décrit pour l'alkylation d'autres trifluoroacétylamides (P.A. Harland, P. Hodge, W. Maughan et E. Wildsmith, Synthesis, 1984, 941).

i5) Les composés de formule générale (I) dans lesquels $R_3$ représente un radical alkylaminométhyl s'obtiennent par hydrolyse des composés de formule générale (I) dans lesquels $R_3$ représente un radical alkylacylaminométhyl. Cette hydrolyse s'effectue de préférence en milieu basique, de préférence avec le concours d'une base alcaline telle que l'hydroxyde de sodium ou l'hydroxyde de potassium, à des températures comprises entre 40 ° C et 100 ° C.

Les composés de formule générale (I), objet de la présente invention, présentent l'avantage d'être très stables et facilement transformables en les azétidines de formule générale (IV), qui sont les composés nécessaires pour synthétiser les composés avec activité antimicrobienne de formule générale (II).

La réaction qui permet la transformation des composés de formule générale (I) en les composés de formule générale (IV), consiste en une hydrogénolyse en utilisant un catalyseur de palladium, de préférence l'hydroxyde de palladium. La réaction s'effectue dans le sein d'un alcool tel que le méthanol ou l'éthanol à une pression d'hydrogène comprise entre $1,11.10^5$ Pa (1,1 atm.) et $20,26.10^5$ Pa (20 atm.) et les températures appropriées oscillent entre 20 ° C et 70 ° C.

Dans les exemples suivants, on indiquera la préparation des nouveaux dérivés selon l'invention. Les exemples ci-après, donnnés à simple titre d'illustration, ne doivent cependant en aucune façon limiter l'étendue de l'invention.

Exemple 1.- Préparation de 3-amino-1-diphénylméthyl-3-méthylazétidine.

a) 1-chloro-2,3-époxy-2-méthylpropane.- On ajoute 534 g (3,0 moles) de N-bromosuccinimide à une suspension de 294 ml (3,0 moles) de chlorure de méthallyle dans 1,5 l d'eau sous agitation vigoureuse et à température ambiante. On agite le mélange pendant 16 heures, on refroidit à 10 ° C, et on ajoute de l'hydroxyde de sodium aqueux à 50% (3 moles) à une vitesse telle que la température se maintienne à environ 25 ° C. On maintient l'agitation pendant 2 heures, on sépare la phase organique inférieure, on sèche avec du sulfate de magnésium (20 g), on évapore et on obtient 266 g (84%) de produit brut. L'extraction de la phase aqueuse avec du chloroforme (250 ml) donne 50 g (12%) additionnels. Le produit peut être utilisé directement pour le stade suivant, mais il est préférable de distiller pour éliminer les dernières traces de succinimide (point d'ébullition 65 ° C 4132 Pa (40 torr)).

Données spectroscopiques :

$^1$H-RMN,$\delta$, ($CDCl_3$): 1,47 (s,3H) ; 2,72 (d,J = 5,0 Hz,
1H) ; 2,79 (d,J = 5,0 Hz, 1H) ; 3,52 (s,2H).

b,c) 1-diphénylméthyl-3-hydroxy-3-méthylazétidine.-

On ajoute 12,5 g (117,3 mmoles) de 1-chloro-2,3-époxy-2-méthylpropane à une solution de 21,5 g (117,3 mmoles) de diphénylméthylamine dissous dans 50 ml de méthanol, on laisse 3 jours à température ambiante et postérieurement 3 jours à reflux. On évapore le méthanol sous pression réduite, on lave le solide résultant avec de l'acétone, on filtre et on obtient 28,8 g (85%) de chlorhydrate de 1-diphénylméthyl-3-hydroxy-3-méthylazétidine de point de fusion 187-197 ° C.

Données spectroscopiques :

$^1$H-RMN,$\delta$, (DMSO-$d_6$): 1,49 (m,3H) ; 3,95 (m,4H) ; 6,06 (m,2H) ; 7,1-7,9 (m,10H) ; 12,2-12,7 (é,1H)

IR (BrK) : 3322, 2587, 1455, 1242, 704 $cm^{-1}$

La base se libère par extraction avec du chloroforme dans une solution d'hydroxyde de sodium à 10% et on obtient le 1-diphénylméthyl-3-hydroxy-3-méthylazétidine avec un rendement de 98%.
Données spectroscopiques :
$^1$H-RMN,δ, ($CDCl_3$): 1,66 (s,3H) ; 3,10 (d,J=8 Hz, 2H) ; 3,33 (d,J=8 Hz, 2H) ; 4,50 (s,1H) ; 7,1-7,7 (m,10H).
j) 1-diphénylméthyl-3-hydroxy-3-méthylazétidine.-
On ajoute goutte à goutte 1,41 g de méthyllithium (64 mmoles) dans 140 ml d'éther anhydre à une solution de 3,05 g (12,8 mmoles) de 1-diphénylméthyl-3-azétidinone dans 61 ml d'éther anhydre, refroidie à 0 °C et on maintient la réaction pendant 2 heures à 0 °C. On ajoute ensuite de l'eau goutte à goutte. On lave la phase organique avec de l'eau, on sèche avec du sulfate de sodium anhydre et on évapore à sec. On dissout le solide résultant dans du méthanol, on ajoute du méthanol saturé d'acide chlorhydrique et on évapore à sec. On lave le solide résultant avec de l'acétone, on filtre et on obtient 3,12 g (84%) de chlorhydrate de 1-diphénylméthyl-3-hydroxy-3-méthylazétidine de point de fusion 187-197 °C.
h) 1-diphénylméthyl-3-méthyl-3-méthylsulfonyloxyazétidine.
On ajoute 5,3 g (46,8 mmoles) de chlorure de méthansulfonyle goutte à goutte à une solution de 7,9 g (31,2 mmoles) de 1-diphénylméthyl-3-hydroxy-3-méthylazétidine dans 70 ml de pyridine refroidie à -20 °C. On maintient la température à -20 °C pendant une heure et on laisse à 4 °C pendant 12 heures. On verse ensuite sur un mélange eau/glace, on filtre le précipité formé et on le lave avec de l'eau. On dissout le solide dans du chlorure de méthylène, on décante l'eau restante, on sèche avec du sulfate de sodium anhydre et on évapore le solvant sous pression réduite. On recristallise le solide obtenu avec un mélange de chlorure de méthylène/heptane et on obtient 7,2 g (70%) de 1-diphénylméthyl-3-méthyl-3-méthylsulfonyloxyazétidine de point de fusion 113-115 °C.
Données spectroscopiques :
$^1$H-RMN,δ, ($CDCl_3$): 1,90 (s,3H) ; 3,00 (s,3H) ; 3,32 (s,4H) ; 4,43 (s,1H) ; 7,1-7,7 (m,10H)
IR (BrK) : 1337, 1165, 941 703 $cm^{-1}$
i) 3-amino-1-diphénylméthyl-3-méthylazétidine.-
On dissout 14,4 g (43,4 mmoles) de 1-diphénylméthyl-3-méthyl-3-méthylsulfonyloxyazétidine dans 100 ml d'une solution de dioxanne saturée d'ammoniac, et on agite à 75-80 °C pendant 20 heures. On évapore à sec, on ajoute de l'eau, on acidifie avec de l'acide acétique, on extrait avec du dichlorométhane, on sèche avec du sulfate dé sodium anhydre, on évapore le solvant et on obtient 8,0 g (73%) de 3-amino-1-diphénylméthyl-3-méthylazétidine de point de fusion 84-6 °C.
Données spectroscopiques :
$^1$H-RMN,δ, ($CDCl_3$) : 1,38 (s,3H); 1,73 (s,2H) ; 2,71 (d,2H,J=8 Hz); 3,10 (d,2H,J=8 Hz); 4,28 (s,1H); 7,0-7,5 (m,10H).
IR : (BrK) : 3400, 1450, 1247, 626 $cm^{-1}$
On dissout 7,75 g (31 mmoles) de 3-amino-1-diphénylméthyl-3-méthylazétidine dans 80 ml de méthanol et on les traite avec de l'éther diéthylique saturé d'acide chlorhydrique jusqu'à pH 5-6.
On évapore à sec de façon exhaustive pour éliminer l'excès d'acide, et on obtient 10,0 g (100%) de dichlorhydrate de 3-amino-1-diphénylméthyl-3-méthylazétidine de point de fusion 128-130 °C.
Données spectroscopiques :
$^1$H-RMN,δ, (DMSO) : 1,70 (s,3H); 3,96 (m,2H); 4,36 (m,2H); 6,63 (m,1H); 7,38-7,69 (m,10H); 9,05 (é,3H); 13,17 (é,1H).
IR : (BrK) : 3400-2300, 1601, 830 $cm^{-1}$

Exemple 2.- Préparation de trans-3-amino-1-diphénylméthyl-2-méthylazétidine

d,e)

tréo-3-bromo-1,2-époxybutane.

On ajoute du $Br_2$ goutte à goutte jusqu'à ce que la solution prenne une légère coloration ($Br_2$ théorique : 45,4 g, 0,284 mole), à une solution de 20,4 g (0,284 mole) de trans-2-butène-1-ol dans 60 ml de chloroforme. On ajoute alors quelques gouttes d'alcool crotilique jusqu'à ce que la solution devienne à nouveau transparente. On maintient 15 minutes à température ambiante, on évapore le solvant et on obtient un résidu liquide obscur. On dissout ce 2,3-dibromo-1-butanol cru dans 140 ml d'éther éthylique et à la solution résultante on ajoute 16 g (0,284 mole) d'hydroxyde de potassium dans 170 ml d'eau. On

agite pendant 2 heures à température ambiante, on sépare les deux couches et on lave la couche organique avec de l'eau. On évapore le solvant, on distille sous vide, et on obtient 24 g (56%) de tréo-3-bromo-1,2-époxybutane de point d'ébullition 55-60 °C à 3332,5 Pa (25 mm de Hg).
Données spectroscopiques :
$^1$H-RMN,δ, ($CDCl_3$) : 1,68 (d,3H,J=7 Hz) ; 2,69 (dd,1H, J=5 Hz, J=2,5 Hz) ; 2,88 (dd,1H,J=5 Hz, J=4 Hz); 3,18 (ddd,1H,J=7 Hz, J=4 Hz, J=2,5 Hz) ; 3,86 (q,1H, J=7 Hz).
f,g)

trans-1-diphénylméthyl-3-hydroxy-2-méthylazétidine.

On maintient sous agitation pendant 80 heures à température ambiante et 72 heures à reflux une solution de tréo-3-bromo-1,2-époxybutane (9,8 g, 64,90 mmoles) et d'aminodiphénylméthane (11,8 g, 64,5 mmoles) dans 70 ml de méthanol. On évapore à sec et on traite le résidu visqueux avec de l'éther et de l'eau. On alcalinise la couche aqueuse avec du carbonate de potassium, on extrait avec de l'éther éthylique et on obtient 9,4 g (61%) de trans-1-diphénylméthyl-3-hydroxy-2-méthylazétidine.
Données spectroscopiques :
$^1$H-RMN,δ, ($CDCl_3$) : 0,75 (d,J=6 Hz); 2,40 (é,1H); 2,56 (t,1H,J=6 Hz); 3,02 (q,1H,J=6 Hz); 3,64 (t,1H, J=6 Hz); 3,87 (quint.,1H,J=6 Hz); 4,34 (s,1H); 7,27 (m,10H).
IR (film) : 3400, 1450, 1156, 749, 702 $cm^{-1}$.
On traite jusqu'à pH 5-6 un échantillon de trans-1-diphénylméthyl-3-hydroxy-2-méthylazétidine dissous dans le méthanol avec de l'éther diéthylique saturé d'acide chlorhydrique. On évapore à sec de façon exhaustive pour éliminer l'excès d'acide et on obtient le chlorhydrate de trans-1-diphénylméthyl-3-hydroxy-2-méthylazétidine de point de fusion 100-103 °C.
h)

trans-1-diphénylméthyl-2-méthyl-3-méthylsulfonyloxy-azétidine.-

On ajoute 50 g (0,495 mole) de triéthylamine à une solution de 77,33 g (0,329 mole) de trans-1-diphénylméthyl-3-hydroxy-2-méthylazétidine dans 600 ml de dichlorométhane et on refroidit à 0 °C. On maintient la température, on additionne goutte à goutte une solution de 50 g (0,437 mole) de chlorure de mésyle et on laisse 24 heures à température ambiante. On lave 2 fois avec de l'eau (300 ml) la solution résultante, on sèche avec du sulfate de sodium anhydre, on évapore et on obtient une huile qui, cristallisée avec de l'éther de pétrole, donne 104,6 g (96%) de trans-1-diphénylméthyl-2-méthyl-3-méthylsulfonyloxyazétidine de point de fusion 68-71 °C.
Données spectroscopiques :
$^1$H-RMN,δ, ($CDCl_3$): 0,63 (d,3H,J=7 Hz); 2,85 (t,1H,J=6 Hz); 2,96 (s,3H); 3,62 (t,2H,J=6 Hz); 4,39 (s,1H); 4,55 (quint.,1H,J=6 Hz); 7,23 (m,10H).
IR (BrK) : 1361, 1339, 1178, 1152, 708 $cm^{-1}$.
i)

trans-3-amino-1-diphénylméthyl-2-méthylazétidine.-

On dissout 31 g (93,65 mmoles) de trans-1-diphénylméthyl-2-méthyl-3-méthylsulfonyloxyazétidine dans un mélange de 150 ml d'isopropanol et de 100 ml d'ammoniaque aqueux à 30%. On chauffe à 70 °C pendant 2-3 heures la solution résultante tout en monitorisant la réaction par chromatographie sur couche mince. On évapore jusqu'à élimination totale de l'isopropanol (environ 1/3 du volume) et on extrait avec de l'éther éthylique et de l'eau. On alcalinise la couche aqueuse, on extrait avec du dichlorométhane et on obtient 10 g de la diamine désirée. On acidifie avec de l'acide acétique dilué (5%) la couche éthérée de la première extraction puis on alcalinise la couche acide avec de l'hydroxyde de sodium, on extrait avec du dichlorométhane et on obtient 6,3 g de diamine, ce qui fait un total obtenu de 16,3 g (70%) de trans-3-amino-1-diphénylméthyl-2-méthylazétidine de point de fusion 68-69 °C.
Données spectroscopiques :
$^1$H-RMN,δ, ($CDCl_3$): 0,64 (d,3H,J=7 Hz); 2,20 (q,1H,J=7 Hz); 2,63 (t,1H,J=7 Hz); 2,90 (quint.,1H,J=7

Hz); 3,50 (t,1H,J = 7 Hz); 4,20 (s,1H); 7,20 (m,10H).
IR (BrK): 3270, 1450, 702 $cm^{-1}$.
On dissout 10,40 g (41,27 mmoles) de trans-3-amino-1-diphénylméthyl-2-méthylazétidine dans 100 ml de méthanol et on les traite avec de l'éther éthylique saturé d'acide chlorhydrique jusqu'à pH 5-6. On sèche ensuite à sec de façon exhaustive pour éliminer l'excès d'acide et on obtient 13,3 g (100%) de dichlorhydrate de trans-3-amino-1-diphénylméthyl-2-méthylazétidine de point de fusion 150-3 ° C.

Exemple 3.- Préparation de 1-diphénylméthyl-3-éthylaminométhyl-3-méthylazétidine

h) 3-cyano-1-diphénylméthyl-3-méthylazétidine
On ajoute 33,1 g (100 mmoles) de 1-diphénylméthyl-3-méthyl-3-méthylsulfonyloxyazétidine à une suspension de cyanure de sodium (11 g, 225 mmoles) dans la diméthylformamide (90 ml) et on agite à 65-70 ° C pendant 6 heures. On refroidit, on verse sur un mélange eau/glace, on filtre, on lave avec de l'eau, on sèche à 50 ° C et on obtient 21,75 g (83%) de 3-cyano-1-diphénylméthyl-3-méthylazétidine de point de fusion 86-88 ° C.
Données spectroscopiques :
$^1$H-RMN,δ, ($CDCl_3$): 1,60 (s,3H); 3,00 (d,2H,J = 7,5 Hz); 3,37 (d,2H,J = 7,5 Hz); 4,30 (s,1H); 7,15 (m,10H)
IR (BrK) : 2843, 1492, 1452, 745, 706 $cm^{-1}$.
i2) 3-aminométhyl-1-diphénylméthyl-3-méthylazétidine
On met en suspension 6,1 g (161 mmoles) de tétrahydrure d'aluminium et de lithium dans 250 ml de tétrahydrofuranne et on ajoute goutte à goutte pendant 1 heure une solution de 21,1 g (80,5 mmoles) de 3-cyano-1-diphénylméthyl-3-méthylazétidine dans 150 ml de tétrahydrofuranne. On maintient la température pendant l'addition entre 30 et 35 ° C et ensuite on agite pendant 12 heures à température ambiante. On détruit l'excès d'hydrure d'aluminium et de lithium avec de l'éthanol, on filtre la fraction minérale insoluble, on élimine le tétrahydrofuranne, on redissout avec du chloroforme, on lave à l'eau, on sèche avec du sulfate de sodium anhydre, on évapore à sec et on obtient 16,1 g (75%) de 3-aminométhyl-1-diphénylméthyl-3-méthylazétidine de point de fusion 46-8 ° C.
Données spectroscopiques :
$^1$H-RMN,δ, ($CDCl_3$): 1,1 (s,3H); 1,3 (é,2H); 2,7-3,0 (m,6H); 4,34 (s,1H); 7,2 (m,10H).
IR (BrK): 1452, 744, 704 $cm^{-1}$.
i3) 1-diphénylméthyl-3-méthyl-3-trifluoroacétylaminométhylazétidine
On ajoute goutte à goutte une solution de 14,8 g (69 mmoles) d'anhydride trifluoroacétique dans 50 ml de chloroforme à une solution de 14,72 g (55,25 mmoles) de 3-aminométhyl-1-diphénylméthyl-3-méthylazétidine dans 100 ml de chloroforme. On maintient la température à 20 ° C pendant l'addition et puis on agite pendant deux heures à 25 ° C. On lave avec de l'eau, avec une solution de bicarbonate de sodium à 10% puis à nouveau avec de l'eau, on sèche avec du sulfate de sodium anhydre, on évapore et on obtient 16,0 g (80%) de 1-diphénylméthyl-3-méthyl-3-trifluoroacétylaminométhylazétidine de point de fusion 127-8 ° C.
Données spectroscopiques :
$^1$H-RMN,δ, ($CDCl_3$): 1,06 (s,3H); 2,85 (d,2H); 3,06 (d,2H); 3,26 (d,2H); 4,30 (s,1H); 7,20 (m,10H); 9,30 (é,1H)
IR (BrK): 2297, 1727, 1175, 1148 $cm^{-1}$.
i4) 1-diphénylméthyl-3-méthyl-3-[(N-éthyl)-trifluoroacétylaminométhyl]-azétidine
On ajoute 0,16 g (3,6 mmoles) d'hydrure de sodium à 55% à une solution de 1,3 g (3,6 mmoles) de 1-diphénylméthyl-3-méthyl-3-trifluoroacétylaminométhylazétidine dans 40 ml de dioxanne et 10 ml de diméthylformamide, et on agite pendant 2 heures à 60-70 ° C. On refroidit la solution à température ambiante, on additionne 0,73 g (4,6 mmoles) d'iodure d'éthyle, on agite 4 heures à 70 ° C, on évapore à sec, on dissout le résidu avec du chloroforme, on lave avec de l'eau, on sèche avec du sulfate de sodium anhydre, on évapore et on obtient 1,1 g (79%) de 1-diphénylméthyl-3-méthyl-3-[(N-éthyl)-trifluoroacétylaminométhyl]-azétidine. On dissout ce composé dans l'éthanol et on ajoute de l'éther éthylique saturé d'acide chlorhydrique. On laisse cristalliser, on filtre et on obtient le chlorhydrate de 1-diphénylméthyl-3-méthyl-3-[(N-éthyl)-trifluoroacétylaminométhyl)-azétidine de point de fusion 191-4 ° C.
Données spectroscopiques :
$^1$H-RMN,δ, (DMSO-$d_6$): 1,15 (m,3H); 1,36 (s,3H); 3,37 (s,2H); 3,72 (m,3H); 4,0 (m,4H); 6,0 (d,1H); 7,6 (m,10H)
IR (BrK) : 1686, 1214, 1149 $cm^{-1}$.
i5) 1-diphénylméthyl-3-éthylaminométhyl-3-méthylazétidine
On agite pendant une heure à 70 ° C 3,9 g (10 mmoles) de 1-diphénylméthyl-3-méthyl-3-[(N-éthyl)-trifluoroacétylaminométhyl]-azétidine dans 20 ml d'hydroxyde de sodium à 5% et 20 ml d'éthanol. On refroidit la solution, on porte à pH 8 avec de l'acide chlorhydrique, on acidifie avec de l'acide acétique, on évapore, on extrait avec du chloroforme, on sèche avec du sulfate de sodium anhydre, on évapore le solvant et on obtient 2,5 g (85%) de 1-diphénylméthyl-3-éthylaminométhyl-3-méthylazétidine.

Données spectroscopiques :

$^{1}$H-RMN,$\delta$, ($CDCl_3$): 1,05 (t,3H); 1,20 (s,3H); 2,4-3,1 (m,9H); 4,31 (s,1H); 7,0-7,6 (m,10H)

IR (film): 2962, 2922, 1452, 753, 743, 703 $cm^{-1}$.

La synthèse des exemples 4 à 17 s'effectue en suivant la méthode des exemples antérieurs. Les données de point de fusion et de spectroscopie infrarouge des exemples 1 à 17 sont présentés dans le tableau 1 et les valeurs correspondantes à la résonance magnétique nucléaire de proton sont présentés dans le tableau 2. Selon un protocole similaire à celui décrit précédemment, on obtient également à titre de composés selon l'invention les dérivés :

- cis-3-amino-1-diphénylméthyl-2-éthylazétidine ;
- 3-amino-2,2-diméthyl-1-diphénylméthylazétidine ;
- (2R,3R)-3-amino-1-diphénylméthyl-2-méthylazétidine ;
- (2S,3S)-3-amino-1-diphénylméthyl-2-méthylazétidine ;
- (2R,3S)-3-amino-1-diphénylméthyl-2-méthylazétidine ;
- (2S,3R)-3-amino-1-diphénylméthyl-2-méthylazétidine.

**TABLEAU 1**

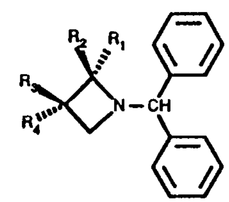

| Exem-ple | $R_1$ | $R_2$ | $R_3$ | $R_4$ | Isomère | Base / Sel | P.f. (ºC) | IR (KBr), $cm^{-1}$ | $[\alpha]_D$ c=0,3 ($CHCl_3$) Base |
|---|---|---|---|---|---|---|---|---|---|
| 1 | H | H | $-NH_2$ | $CH_3$ | - | Base | 84-86 | 3400, 1450, 1247, 626 | - |
| 1 | H | H | $-NH_2$ | $CH_3$ | - | 2HCl | 128-130 | 3400-2300, 1601, 830 | - |
| 2 | $CH_3$ | H | $-NH_2$ | H | trans | Base | 68-69 | 3270, 1450, 702 | - |
| 2 | $CH_3$ | H | $-NH_2$ | H | trans | 2HCl | 150-153 | 3400-2300, 1453, 704 | - |
| 3 | H | H | $-CH_2NHCH_2CH_3$ | $CH_3$ | - | Base | (Huile) | 2962, 2922, 1452, 753, 743, 703 | - |
| 4 | H | H | $-CH_2N(Et)COCF_3$ | $CH_3$ | - | HCl | 191-194 | 1686, 1214, 1149 | - |
| 5 | H | H | $-CH_2NHCOCF_3$ | $CH_3$ | - | Base | 127-128 | 2297, 1727, 1175, 1148 | - |
| 6 | H | H | $-CH_2NH_2$ | $CH_3$ | - | Base | 46-48 | 1452, 744, 704 | - |
| 7 | H | H | $-NHCH_3$ | $CH_3$ | - | Base | 62-63 | 3293, 2820, 1450, 705 | - |
| 8 | H | H | $-N(CH_3)_2$ | $CH_3$ | - | Base | 53-54 | 2824, 1235, 706 | - |
| 8 | H | H | $-N(CH_3)_2$ | $CH_3$ | - | 2HCl | 190-192 | 2361, 1453, 1422, 756, 706 | - |
| 9 | $CH_3$ | H | $-NHCH_3$ | H | trans | Base | 93-95 | 1960, 2920, 1470, 705 | - |
| 10 | $CH_3$ | H | $-N(CH_3)_2$ | H | trans | 2HCl | 149-152 | 3600-3100, 1457, 752, 706 | - |
| 11 | $CH_3$ | H | $-CH_2NH_2$ | H | trans | Base | 84-87 | 3402, 2870, 1453, 704 | - |
| 12 | $CH_3$ | H | $-CH_2NHCOCF_3$ | H | trans | Base | 122-125 | 3292, 1705, 1180, 703 | - |
| 13 | $CH_3$ | H | $-CH_2N(Et)COCF_3$ | H | trans | HCl | 95-101 | 1689, 1456, 1187, 705 | - |
| 14 | H | $CH_3$ | $-NH_2$ | H | cis | 2HCl | 135-138 | 3350, 1492, 1451, 704 | - |
| 15 | $CH_3$ | H | $-NH_2$ | $CH_3$ | r-3-amino--trans-2- | 2HCl | 172-174 | 3500-2200, 1457, 1390, 753, 704 | - |
| 16 | H | H | $-NHCOCF_3$ | $CH_3$ | - | HCl | (Huile) | 3300, 1784, 1700, 1162, 704(film) | - |
| 17 | H | H | $-CH_2NHCOCH_3$ | $CH_3$ | - | Base | (Huile) | 3300, 1656, 1556, 788, 704(film) | - |

**TABLEAU 1**

(Suite)

| Exemple | $R_1$ | $R_2$ | $R_3$ | $R_4$ | Isomère | Base / Sel | P.f. (ºC) | IR (KBr), $cm^{-1}$ | $[\alpha]_D$ c=0,3 ($CHCl_3$) Base |
|---|---|---|---|---|---|---|---|---|---|
| 18 | H | $CH_3CH_2$ | $-NH_2$ | H | cis | Base | 86,6 Pa P.eb. 200-235 (0,65 torr) | 3380, 3312, 1600, 1492, 1451(film) | - |
| 18 | H | $CH_3CH_2$ | $-NH_2$ | H | cis | 2HCl | 123-125 | 3412, 2931, 1450, 750, 700 | - |
| 19 | $CH_3$ | $CH_3$ | $-NH_2$ | H | - | Base | 103-106 | 3368, 3000, 1587, 1493, 1450 | - |
| 19 | $CH_3$ | $CH_3$ | $-NH_2$ | H | - | 2HCl | 150-152 | 3412, 2943, 1450, 1122, 743, 700 | - |
| 20 | H | $CH_3$ | $-NH_2$ | H | 2R,3R | 2HCl | 128-130 | 3348, 1492, 1450, 703 | +74,0 |
| 21 | $CH_3$ | H | H | $-NH_2$ | 2S,3S | 2HCl | 130-132 | 3348, 1492, 1450, 703 | -73,3 |
| 22 | H | $CH_3$ | H | $-NH_2$ | 2R,3S | 2HCl | 151-153 | 3400-2300, 1453, 704 | +112,3 |
| 23 | $CH_3$ | H | $-NH_2$ | H | 2S,3R | 2HCl | 152-153 | 3400-2300, 1453, 704 | -110,3 |

TABLEAU 2

| Exemple | $^1H$-RMN, δ, ($CDCl_3$) ou ($DMSO-D_6$)* |
|---|---|
| 1 | 1,38 (s,3H); 1,73 (s,2H); 2,71 (d,2H,J=8 Hz); 3,10 (d,2H,J=8 Hz); 4,28(s,1H); 7,0-7,5 (m,10 H) |
| 1.2 HCl | *1,70 (5,3H); 3,96 (m,2H); 6,63 (m,1H); 7,38-7,69 (m,10H); 9,05 (é,3H); 13,17 (é,1H) |
| 2 | 0,64 (d,3H,J=7Hz); 2,20 (q,1H,J=7z); 2,63 (t,1H,J=7Hz); 2,90 (quint., 1H,J=7Hz); 3,50 (t,1,J=7Hz); 4,20 (s,1H); 7,20 (m,10H) |
| 3 | 1,05 (t,3H); 1,20 (s,3H); 2,4-3,1 (m,9H); 4,31 (s,1H); 7,0-7,6 (m,10H) |
| 4.HCl | *1,15 (m,3H); 1,36 (s,3H); 3,37 (s,2H); 3,72 (m,3H), 4,0 (m,4H); 6,0 (d,1H); 7,6 (m,10) |
| 5 | 1,06 (s,3H); 2,85 (d,2H); 3,06 (d,2H); 3,26 (d,2H); 4,30 (s,1H); 7,20 (m,10H); 9,3 (é,1H) |
| 6 | 1,1 (s,3H); 1,30 (é,2H); 2,7-3,0 (m,6H); 4,34 (s,1H); 7,2 (m,10H) |
| 7 | 1,3 (s,1H); 1,4 (s,3H); 2,2 (s,3H); 2,60 (d,2H); 3,0 (d,2H); 4,2 (s,1H); 7,25 (m,10H) |
| 8 | 1,28 (s,3H); 2,07 (s,6H); 2,88 (d,2H,J=7Hz); 3,05 (d,2H,J=7Hz); 4,43 (s,1H); 7,30 (m,10H) |
| 9 | 0,8 (d,3H,J=6Hz); 1,2 (é,1H); 2,28 (s,3H); 2,29 (m,1H); 2,85 (m,2H); 3,5 (m,1H); 4,25 (s,1H); 7,25 (m,10H) |

**TABLEAU 2**

(Suite)

| Exemple | $^1H$-RMN, δ, ($CDCl_3$) |
|---|---|
| 10 | 0,75 (d,3H,J=6Hz); 2,05 (s,6H); 2,35 (q,1H,J=6,5Hz); 2,6 (t,1H,J=6,5Hz); 3,15 (quint., 1H,J=6,5Hz); 3,5 (t,1H,J=6,5z); 4,4 (s,1H); 7,3 (m,10H) |
| 11 | 0,75 (d,3H); 1,36 (é,2H); 2,10 (sext.,1H); 2,42 (t,1H); 2,71 (d,2H); 2,97 (quint.,1H); 3,45 (t,1H); 4,3 (s,1H); 7,3 (m,10H) |
| 12 | 0,85 (d,3H); 2,25 (sext.,1H); 2,55 (t,1H); 3,16 (quint.,1H); 3,50 (m,3H); 4,41 (s,1H); 6,98 (é,1H); 7,33 (m,10H) |
| 13.HCl | 1,0 (m,5H); 2,8 (m,1H); 3,2 (q,2H); 3,6 (d,2H); 3,95 (m,1H); 4,5 (m,1H); 5,65 (d,1H); 7,21-7,75 (m,10H); 12,17 (é,1H) |
| 14 | 0,63 (d,3H,J=6Hz); 1,64 (é,2H); 3,09 (m,2H); 3,35 (m,2H); 4,34 (s,1H); 7,27 (m,10H) |
| 15 | 0,53 (d,3H,J=6,5Hz); 1,26 (s,3H); 1,51 (é,2H); 2,41 (d,1H,J=7Hz); 2,84 (q,1H,J=7Hz); 3,25 (d,1H,J=7Hz); 4,27 (s,1H); 7,26 (m,10H) |
| 16 | 1,56 (s,3H); 3,18(s,4H); 4,38 (s,1H); 7,25 (m,10H); 9,2 (é,1H) |
| 17 | 1,05 (s,3H); 1,91 (s,3H); 2,70 (d,2H,J=7,6Hz); 2,96 (d,2H,J=7,6Hz, 3,20 (d,2H,J=5Hz); 4,24 (s,1H); 6,25 (m,10H); 9,2 (é,1H) |

**TABLEAU 2**

**(Suite)**

| Exemple | $^1H$-RMN, δ, ($CDCl_3$) |
|---|---|
| 18 | 0,54 (m,3H); 1,45 (m,2H); 1,71 (é,2H); 3,04 (m,3H); 3,46 (dt,J=6,2Hz, J'=2,3Hz, 1H); 4,29 (s,3H); 7,05-7,42 (m,10H) |
| 19 | 1,00 (s,6H); 1,51 (é,2H); 2,51 (t,J=6,1Hz, 1H); 3,26 (m,2H); 4,54 (s,1H); 7,10-7,55 (m,10H) |
| 20 | 0,63 (d,3H,J=6Hz); 1,64 (s,2H); 3,09 (m,2H); 3,35 (m,2H); 4,34 (s,1H); 7,27 (m,10H) |
| 21 | 0,63 (d,3H,J=6Hz); 1,64 (s,2H); 3,09 (m,2H); 3,35 (m,2H); 4,34 (s,1H); 7,27 (m,10H) |
| 22 | 0,64 (d,3H,J=7Hz); 2,20 (q,1H,J=7Hz); 2,63 (t,1H,J=7Hz); 2,90 (quint.,1H,J=7Hz); 3,50 (t,1H,J=7Hz); 4,20 (s,1H); 7,20 (m,10H) |
| 23 | 0,64 (d,3H,J=7Hz); 2,20 (q,1H,J=7Hz); 2,63 (t,1H,J=7Hz); 2,90 (quint.,1H,J=7Hz); 3,50 (t,1H,J=7Hz); 4,20 (s,1H); 7,20 (m,10H) |

Exemple 1 A. - Préparation du dichlorhydrate de 3-amino-3-méthylazétidine

On dissout 10 g (31 mmoles) de dichlorhydrate de 3-amino-1-diphénylméthyl-3-méthylazétidine dans 120 ml de méthanol et on ajoute 2 g de $Pd(OH)_2$ i.e. 20% en poids. On maintient pendant 12 heures avec

de l'hydrogène sous pression (15,2.$10^5$ Pa (15 atm.)), on filtre le catalyseur, on évapore le solvant, on élimine le diphénylméthane qui procède de la réaction et on lave avec du benzène et du tétrachlorure de carbone. On recristallise le résidu résultant avec du méthanol et on obtient 3,85 g (78%) de dichlorhydrate de 3-amino-3-méthylazétidine de point de fusion 196-9 ° C.

Données spectroscopiques :

$^1$H-RMN,$\delta$, (DMSO-$d_6$): 1,66 (s,3H); 3,81 (d,2H,J = 10,5 Hz); 4,31 (d,2H,J = 10,5 Hz); 9,32 (é,5H).

IR (BrK): 3300-2300, 1575, 1515, 1232 $cm^{-1}$.

La synthèse des exemples 2A à 16A s'effectue en suivant la méthode de l'exemple 1A. Les résultats correspondants aux points de fusion, à la spectroscopie infrarouge et à la résonance magnétique nucléaire de proton sont présentés dans les tableaux 3 et 4.

TABLEAU 3

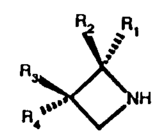

| Exemple | $R_1$ | $R_2$ | $R_3$ | $R_4$ | Isomère | Base Sel | P.f. (ºC) | IR (KBr), $cm^{-1}$ |
|---|---|---|---|---|---|---|---|---|
| 1A | H | H | $-NH_2$ | $CH_3$ | - | 2HCl | 196-199 | 3300-2300, 1575, 1515, 1232 |
| 2A | $CH_3$ | H | $-NH_2$ | H | trans | 2HCl | 165-168 | 3500-2100, 1561, 1451, 1365, 1043 |
| 4A | H | H | $-CH_2N(Et)COCF_3$ | $CH_3$ | - | HCl | 120-123 | 2960, 1688, 1270, 1190, 1130 |
| 5A | H | H | $-CH_2NHCOCF_3$ | $CH_3$ | - | HCl | 187-192 | 3327, 2939, 1729, 1209, 1186, 1157 |
| 6A | H | H | $-CH_2NH_2$ | $CH_3$ | - | 2HCl | 223-226 | 2980, 2940, 1580, 1500 |
| 8A | H | H | $-N(CH_3)_2$ | $CH_3$ | - | 2HCl | 185-186 | 3120, 2870, 1458, 1190 |
| 9A | $CH_3$ | H | $-NHCH_3$ | H | trans | 2HCl | (Huile) | 2925, 1618, 1450, 1075 |
| 10A | $CH_3$ | H | $-N(CH_3)_2$ | H | trans | 2HCl | 170-174 | 3300-2300, 1473, 1382, 1252 |
| 12A | $CH_3$ | H | $-CH_2NHCOCF_3$ | H | trans | HCl | 130-132 | 3068, 1728, 1215, 1157 |
| 13A | $CH_3$ | H | $-CH_2N(Et)COCF_3$ | H | trans | HCl | 180-182 | 2900, 1685, 1222, 1106 |
| 14A | H | $CH_3$ | $-NH_2$ | H | cis | 2HCl | 181-183 | 3300-2200, 1561, 1338, 1188, 1051 |
| 15A | $CH_3$ | H | $-NH_2$ | $CH_3$ | 3-r-amino--2-trans | 2HCl | 180-183 | 3300-2300, 1596, 1554, 1159 |
| 16A | H | H | $-NHCOCF_3$ | $CH_3$ | - | HCl | (Huile) | 3600-2500, 1713, 1555, 1450, 1187 |
| 17A | $CH_3$ | H | $-N(CH_3)COCF_3$ | H | trans | HCl | 133-134 | 2900, 1715, 1270, 1215, 1110 |
| 17B | H | H | $-N(CH_3)COCF_3$ | $CH_3$ | - | HCl | 175-179 | 3480, 2900, 1686, 1153 |

**TABLEAU 3**

(Suite)

| Exemple | $R_1$ | $R_2$ | $R_3$ | $R_4$ | Isomère | Base/Sel | P.f. (ºC) | IR (KBr), $cm^{-1}$ |
|---|---|---|---|---|---|---|---|---|
| 18A | H | $CH_3CH_2$ | $-NH_2$ | H | cis | 2HCl | (Huile) | 3600-2500, 1600, 1462, 1325 |
| 19A | $CH_3$ | $CH_3$ | $-NH_2$ | H | - | 2HCl | 168-171 | 3431, 2968, 1581, 1543, 1512 |
| 20A | H | $CH_3$ | $-NH_2$ | H | 2R,3R | 2HCl | 180-184 | 3300-2200, 1561, 1340 |
| 21A | $CH_3$ | H | H | $-NH_2$ | 2S,3S | 2HCl | 181-186 | 3295-2200, 1561, 1340 |
| 22A | H | $CH_3$ | H | $-NH_2$ | 2R,3S | 2HCl | 164-166 | 3500-2100, 1561, 1451, 1365, 1043 |
| 23A | $CH_3$ | H | $-NH_2$ | H | 2S,3R | 2HCl | 163-165 | 3500-2100, 1561, 1451, 1365, 1043 |

**TABLEAU 4**

| Exemple | $^1$H-RMN, δ, (DMSO-$D_6$) |
|---|---|
| 1A | 1,66 (s,3H); 3,81 (d,2H,J=10,5Hz); 4,31 (d,2H,J=10,8Hz); 9,32 (é,5H) |
| 2A | 1,51 (d,3H,J=7Hz); 3,92 (m,3H); 4,60 (m,1H); 9,20 (é,5H) |
| 4A | 1,15 (t,3H); 1,25 (s,3H); 3,4 (q,2H); 3,57 (s,2H); 3,6 (d,2H,J=9Hz); 3,92 (d,2H,J=9Hz); 9,60 (é,2H) |
| 5A | 1,24 (s,3H); 3,38 (s,2H); 3,54 (m,2H); 3,83 (m,2H); 9,39 (é,1H); 9,78 (é,2H) |
| 6A | 1,35 (s,3H); 3,13 (s,2H); 3,58 (d,2H,J=11Hz); 3,91 (d,2H,J=11Hz); 8,77 (é,5H) |
| 8A | 1,58 (s,3H); 2,50 (s,6H); 3,65 d,2H,J=8Hz); 4,34 (d,2H,J=8Hz); 9,95 (é,3H) |
| 9A | 1,6 (d,3H); 2,6 (s,3H); 3,5 (m,1H); 4,0 (m,2H); 4,8 (m,1H); 9,7 (é,2H); 10,2 (é,2H) |
| 10A | 1,51 (d,3H); 2,70 (s,6H); 4,04 (m,3H); 4,60(m,1H); 9,95 (é,3H) |
| 12A | 1,00 (d,3H); 3,30 (m,6H); 9,40 (m,3H) |
| 13A | 1,12 (t,3H); 1,15 (d,3H); 3,41 (m,8H); 9,9 (é,1H); 10,1 (é,1H) |
| 14A | 1,59 (d,3H); 4,09 (m,3H); 4,59 (m,1H); 9,21 (é,5H) |
| 15A | 1,38 (d,3H); 1,53 (s,3H); 3,57 (d,1H,J=9,5Hz); 4,13 (d,1H,J=9,5Hz); 4,67(m,1H); 9,21 (é,5H) |
| 16A | 1,47 (s,3H); 3,70 (m,4H); 9,45 (é,3H) |
| 17A | 1,48 (d,3H); 3,42 (s,3H); 4,0 (m,2H); 4,65 (m,2H); 9,51 (é,2H) |
| 17B | 1,60 (s,3H); 2,96 (s,3H); 3,70 (d,2H,J=11Hz); 4,19 (d,2H,J=11Hz); 9,56 (é,2H) |

**TABLEAU 4**

(Suite)

| Exemple | $^1H$-RMN, δ, ($DMSO-D_6$) |
|---|---|
| 18A | 0,92 (é,3H); 2,07 (m,2H); 3,42 (m,1H); 4,15 (m,3H); 9,26 (m,5H) |
| 19A | 1,59 (s,3H); 1,68 (s,3H); 3,80-4,20 (m,3H); 9,16 (m,5H) |
| 20A | 1,59 (d,3H); 4,09 (m,3H); 4,59 (m,1H); 9,21 (é,5H) |
| 21A | 1,59 (d,3H); 4,09 (m,3H); 4,59 (m,1H); 9,21 (é,5H) |
| 22A | 1,51 (d,3H,J=7Hz); 3,92 (m,3H); 4,60 (m,1H); 9,20 (é,5H) |
| 23A | 1,51 (d,3H,J=7Hz); 3,92 (m,3H); 4,60 (m,1H); 9,20 (é,5H) |

## Revendications

**1.** Azétidines caractérisées en ce qu'elles répondent à la formule générale (I)

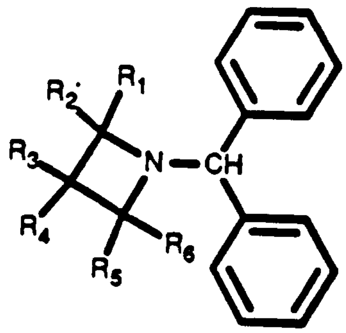

(I)

dans laquelle

$R_3$ représente un radical amino, un radical alkylamino, un radical dialkylamino, un radical cycloalkylamino, un radical acylamino, un radical alkylacylamino, un radical aminométhyle, un radical alkylaminométhyle, un radical acylaminométhyle ou un radical alkylacylaminométhyle, dans lesquels chaque fragment acyle peut être substitué par un ou plusieurs atomes d'halogène, en particulier le fluor ;

$R_1$, $R_2$, $R_4$, $R_5$ et $R_6$ représentent un atome d'hydrogène ou un radical alkyle inférieur, avec la condition que au moins un d'eux représente un radical alkyle inférieur, ainsi que leurs sels.

**2.** Les composés répondant à la formule générale (I), selon la revendication 1, sélectionnés dans le groupe suivant :

- 3-amino-1-diphénylméthyl-3-méthylazétidine ;
- trans-3-amino-1-diphénylméthyl-2-méthylazétidine ;
- 1-diphénylméthyl-3-éthylaminométhyl-3-méthylazétidine ;
- 1-diphénylméthyl-3-[N-(éthyl)-trifluoroacétylaminométhyl]-3-méthylazétidine ;
- 1-diphénylméthyl-3-méthyl-3-trifluoroacétylaminométhylazétidine ;
- 3-aminométhyl-1-diphénylméthyl-3-méthylazétidine ;
- 1-diphénylméthyl-3-méthyl-3-méthylaminoazétidine ;
- 3-diméthylamino-1-diphénylméthyl-3-méthylazétidine ;
- trans-1-diphénylméthyl-2-méthyl-3-méthylaminoazétidine ;
- trans-3-diméthylamino-1-diphénylméthyl-2-méthylazétidine ;
- trans-3-aminométhyl-1-diphénylméthyl-2-méthylazétidine ;
- trans-1-diphénylméthyl-2-méthyl-3-trifluoroacétylaminométhylazétidine ;
- trans-1-diphénylméthyl-3-[N-(éthyl)-trifluoroacétylaminométhyl]-2-méthylazétidine ;
- cis-3-amino-1-diphénylméthyl-2-méthylazétidine ;
- r-3-amino-3,trans-2-diméthyl-1-diphénylméthyazétidine ;
- 1-diphénylméthyl-3-méthyl-3-trifluoroacétylaminoazétidine ;
- 3-acétylaminométhyl-1-diphénylméthyl-3-méthylazétidine ;
- cis-3-amino-1-diphénylméthyl-2-éthylazétidine ;
- 3-amino-2,2-diméthyl-1-diphénylméthylazétidine ;
- (2R,3R)-3-amino-1-diphénylméthyl-2-méthylazétidine ;
- (2S,3S)-3-amino-1-diphénylméthyl-2-méthylazétidine ;
- (2R,3S)-3-amino-1-diphénylméthyl-2-méthylazétidine ;
- (2S,3R)-3-amino-1-diphénylméthyl-2-méthylazétidine.

**3.** Procédé de préparation des composés de formule générale (I) selon l'une des revendications 1 et 2, caractérisé en ce que l'on effectue l'une des opérations suivantes :

* réaction de substitution d'un composé de formule générale (XII)

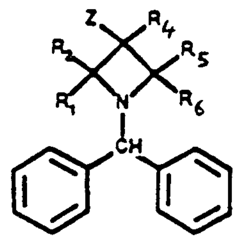

(XII)

dans laquelle Z représente un radical méthylsulfonyloxy ou p-toluènesulfonyloxy, avec de l'ammoniaque, une alkylamine, une dialkylamine ou une cycloalkylamine ;

* réduction d'un composé de formule générale (XII) dans laquelle Z représente un groupe cyano, notamment à l'aide d'un hydrure métallique ;
* acylation d'un composé de formule générale (I) dans laquelle $R_3$ représente un radical amino, alkylamino ou aminométhyle avec un chlorure ou un anhydride d'acide carboxylique ;
* alkylation des composés de formule générale (I) dans laquelle $R_3$ représente un radical acylaminométhyle par réaction avec un halogénure d'alkyle ;
* hydrolyse des composés de formule générale (I) dans laquelle $R_3$ représente un radical alkylacylaminométhyle, de préférence en milieu basique.

**4.** Procédé selon la revendication 3, caractérisé en ce que les composés de formule générale (XII) dans lesquels Z représente un radical méthylsulfonyloxy ou un radical p-toluènesulfonyloxy, sont préparés par réaction des composés de formule générale (XI)

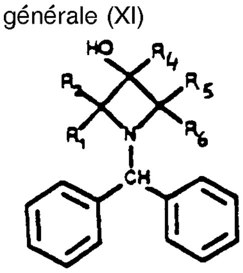

(XI)

avec du chlorure de l'acide méthansulfonique ou du chlorure de l'acide p-toluènesulfonique.

**5.** Procédé selon la revendication 3, caractérisé en ce que les composés de formule générale (XII) dans lesquels Z représente un radical cyano, sont préparés par réaction des composés de formule générale (XII) dans lesquels Z représente un radical méthylsulfonyloxy ou un radical p-toluènesulfonyloxy avec du cyanure de sodium.

**6.** L'utilisation des composés de formule générale (I), selon les revendications 1 et 2, comme intermédiaire pour la préparation de composés pharmaceutiquement utiles.

**7.** L'utilisation des composés de formule générale (I), selon la revendication 6, comme intermédiaires pour la préparation de dérivés de quinolones, naphtyridinones, pyridobenzoxazines, isothiazoloquinoléines, thiazetoquinolones, benzoquinolizines, benzothiazoloquinolones, pyridobenzothiazines, benzoxazoloquinolones et époxyméthanothiazoloquinolones.

## Claims

**1.** Azetidines, characterized in that they correspond to the general formula (I)

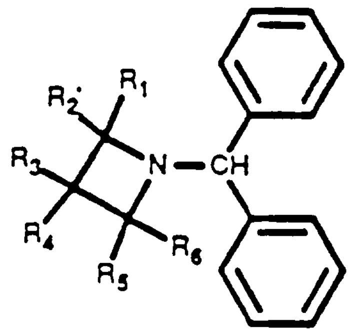

(I)

in which $R_3$ represents an amino radical, an alkylamino radical, a dialkylamino radical, a cycloalkylamino radical, an acylamino radical, an alkylacylamino radical, an aminomethyl radical, an alkylaminomethyl radical, an acylaminomethyl radical or an alkylacylaminomethyl radical, in which radicals each acyl fragment may be substituted with one or more halogen, especially fluorine, atoms; $R_1$, $R_2$, $R_4$, $R_5$ and $R_6$ represent a hydrogen atom or a lower alkyl radical, with the proviso that at least one of them represents a lower alkyl radical, and also their salts.

**2.** The compounds corresponding to the general formula (I) according to Claim 1, selected from the following group:

- 3-amino-1-diphenylmethyl-3-methylazetidine;
- trans-3-amino-1-diphenylmethyl-2-methylazetidine;
- 1-diphenylmethyl-3-ethylaminomethyl-3-methylazetidine;
- 1-diphenylmethyl-3-[N-(ethyl)trifluoroacetylaminomethyl]-3-methylazetidine;
- 1-diphenylmethyl-3-methyl-3-trifluoroacetylaminomethylazetidine;
- 3-aminomethyl-1-diphenylmethyl-3-methylazetidine;
- 1-diphenylmethyl-3-methyl-3-methylaminoazetidine;
- 3-dimethylamino-1-diphenylmethyl-3-methylazetidine;
- trans-1-diphenylmethyl-2-methyl-3-methylaminoazetidine;
- trans-3-dimethylamino-1-diphenylmethyl-2-methylazetidine;
- trans-3-aminomethyl-1-diphenylmethyl-2-methylazetidine;
- trans-1-diphenylmethyl-2-methyl-3-trifluoroacetylaminomethylazetidine;
- trans-1-diphenylmethyl-3-[N-(ethyl)trifluoroacetylaminomethyl]-2-methylazetidine;
- cis-3-amino-1-diphenylmethyl-2-methylazetidine;
- r-3-amino-3,trans-2-dimethyl-1-diphenylmethylazetidine;
- 1-diphenylmethyl-3-methyl-3-trifluoroacetylaminoazetidine;
- 3-acetylaminomethyl-1-diphenylmethyl-3-methylazetidine;
- cis-3-amino-1-diphenylmethyl-2-ethylazetidine;
- 3-amino-2,2-dimethyl-1-diphenylmethylazetidine;
- (2R,3R)-3-amino-1-diphenylmethyl-2-methylazetidine;
- (2S,3S)-3-amino-1-diphenylmethyl-2-methylazetidine;
- (2R,3S)-3-amino-1-diphenylmethyl-2-methylazetidine;
- (2S,3R)-3-amino-1-diphenylmethyl-2-methylazetidine.

**3.** Process for preparing the compounds of general formula (I) according to one of Claims 1 and 2, characterized in that one of the following operations is performed:

* substitution reaction of a compound of general formula (XII)

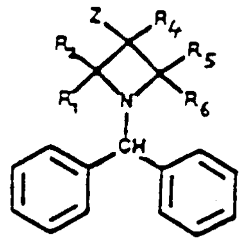

( XII )

in which Z represents a methylsulphonyloxy or p-toluenesulphonyloxy radical with ammonia solution, an alkylamine, a dialkylamine or a cycloalkylamine;

* reduction of a compound of general formula (XII) in which Z represents a cyano group, in particular by means of a metal hydride;
* acylation of a compound of general formula (I) in which $R_3$ represents an amino, alkylamino or aminomethyl radical with a carboxylic acid chloride or anhydride;
* alkylation of compounds of general formula (I) in which $R_3$ represents an acylaminomethyl radical by reaction with an alkyl halide;
* hydrolysis of the compounds of general formula (I) in which $R_3$ represents an alkylacylaminomethyl radical, preferably in a basic medium.

**4.** Process according to Claim 3, characterized in that the compounds of general formula (XII) in which Z represents a methylsulphonyloxy radical or a p-toluenesulphonyloxy radical are prepared by reaction of the compounds of general formula (XI)

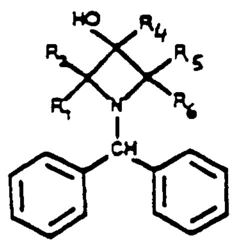

( XI )

with methanesulphonyl chloride or p-toluenesulphonyl chloride.

**5.** Process according to Claim 3, characterized in that the compounds of general formula (XII) in which Z represents a cyano radical are prepared by reaction of the compounds of general formula (XII) in which Z represents a methylsulphonyloxy radical or a p-toluenesulphonyloxy radical with sodium cyanide.

**6.** The use of the compounds of general formula (I) according to Claims 1 and 2 as an intermediate for the preparation of pharmaceutically useful compounds.

**7.** The use of the compounds of general formula (I) according to Claim 6 as intermediates for the preparation of quinolone, naphthyridinone, pyridobenzoxazine, isothiazoloqinoline, thiazetoquinolone, benzoquinolizine, benzothiazoloquinolone, pyridobenzothiazine, benzoxazoloquinolone and epoxymethanothiazoloquinolone derivatives.

**Patentansprüche**

**1.** Azetidine, dadurch gekennzeichnet, daß sie die allgemeinen Formel (I) haben

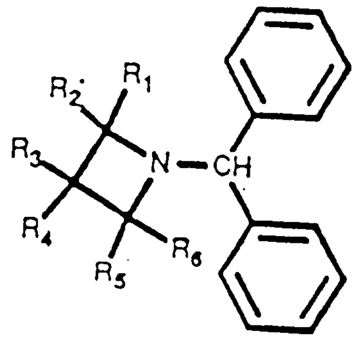

(I)

worin bedeuten:

$R_3$ einen Amino-, Alkylamino-, Dialkylamino-, Cycloalkylamino-, Acylamino-, Alkylacylamino-, Aminomethyl-, Alkylaminomethyl-, Acylaminomethyl- oder Alkylacylaminomethyl-Rest, von denen jeder acyclische Teil (Fragment) substituiert sein kann durch ein oder mehr Halogenatome, insbesondere Fluoratome;

$R_1$, $R_2$, $R_4$, $R_5$ und $R_6$ ein Wasserstoffatom oder einen niederen Alkylrest, mit der Maßgabe, daß mindestens einer von ihnen einen niederen Alkylrest darstellt, sowie ihre Salze.

**2.** Verbindungen der allgemeinen Formel (I) nach Anspruch 1, ausgewählt aus der folgenden Gruppe:
- 3-Amino-1-diphenylmethyl-3-methylazetidin ;
- trans-3-Amino-1-diphenylmethyl-2-methylazetidin ;
- 1-Diphenylmethyl-3-ethylaminomethyl-3-methylazetidin ;
- 1-Diphenylmethyl-3-[N-(ethyl)-trifluoroacetylaminomethyl]-3-methylazetidin ;
- 1-Diphenylmethyl-3-methyl-3-trifluoroacetylaminomethylazetidin ;
- 3-Aminomethyl-1-diphenylmethyl-3-methylazetidin ;
- 1-Diphenylmethyl-3-methyl-3-methylaminoazetidin ;
- 3-Dimethylamino-1-diphenylmethyl-3-methylazetidin ;
- trans-1-Diphenylmethyl-2-methyl-3-methylaminoazetidin ;
- trans-3-Dimethylamino-1-diphenylmethyl-2-methylazetidin ;
- trans-3-Aminomethyl-1-diphenylmethyl-2-methylazetidin ;
- trans-1-Diphenylmethyl-2-methyl-3-trifluoroacetylaminomethylazetidin ;
- trans-1-Diphenylmethyl-3-[N-(ethyl)-trifluoroacetylaminomethyl]-2-methylazetidin ;
- cis-3-Amino-1-diphenylmethyl-2-methylazetidin ;
- r-3-Amino-3,trans-2-dimethyl-1-diphenylmethylazetidin ;
- 1-Diphenylmethyl-3-methyl-3-trifluoroacetylaminoazetidin ;
- 3-Acetylaminomethyl-1-diphenylmethyl-3-methylazetidin ;
- cis-3-Amino-1-diphenylmethyl-2-ethylazetidin ;
- 3-Amino-2,2-dimethyl-1-diphenylmethylazetidin ;
- (2R,3R)-3-Amino-1-diphenylmethyl-2-methylazetidin ;
- (2S,3S)-3-Amino-1-diphenylmethyl-2-methylazetidin ; (2R,3S)-3-Amino-1-diphenylmethyl-2-methylazetidin ;
- (2S,3R)-3-Amino-1-diphenylmethyl-2-methylazetidin .

**3.** Verfahren zur Herstellung der Verbindungen der allgemeinen Formel (I) nach einem der Ansprüche 1 und 2, dadurch gekennzeichnet, daß man eine der folgenden Operationen durchführt:
* Substitutionsreaktion bei einer Verbindung der allgemeinen Formel (XII)

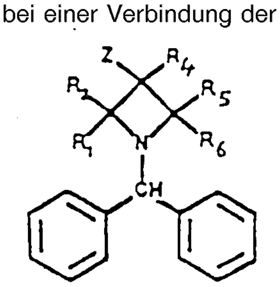

(XII)

worin Z steht für einen Methylsulfonyloxy- oder p-Toluolsulfonyloxy-Rest, mit Ammoniak, einem Alkylamin, einem Dialkylamin oder einem Cycloalkylamin;

* Reduktion einer Verbindung der allgemeinen Formel (XII), worin Z für eine Cyanogruppe steht, insbesondere mit einem Metallhydrid;
* Acylierung einer Verbindung der allgemeinen Formel (I), in der $R_3$ für einen Amino-, Alkylamino- oder Aminomethylrest steht, mit einem Carbonsäurechlorid oder Carbonsäureanhydrid;
* Alkylierung der Verbindungen der allgemeinen Formel (I), in der $R_3$ für einen Acylaminomethyl-Rest steht, durch Umsetzung mit einem Alkylhalogenid;
* Hydrolyse der Verbindungen der allgemeinen Formel (I), in der $R_3$ für einen Alkylacylaminomethylrest steht, vorzugsweise in basischem Medium.

**4.** Verfahren nach Anspruch 3, dadurch gekennzeichnet, daß die Verbindungen der allgemeinen Formel (XII), in denen Z für einen Methylsulfonyloxy- oder p-Toluolsulfonyloxy-Rest steht, hergestellt werden durch Umsetzung der Verbindungen der allgemeinen Formel (XI)

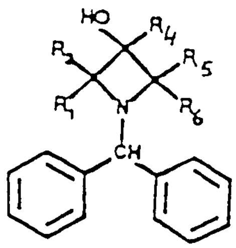

(XI)

mit Methansulfonsäurechlorid oder p-Toluolsulfonsäurechlorid.

**5.** Verfahren nach Anspruch 3, dadurch gekennzeichnet, daß die Verbindungen der allgemeinen Formel (XII), in denen Z einen Cyanorest darstellt, hergestellt werden durch Umsetzung der Verbindungen der allgemeinen Formel (XII), in denen Z einen Methylsulfonyloxy- oder p-Toluolsulfonyloxy-Rest darstellt, mit Natriumcyanid.

**6.** Verwendung der Verbindungen der allgemeinen Formel (I) nach den Ansprüchen 1 und 2 als Zwischenprodukt zur Herstellung von pharmazeutisch verwendbaren (nützlichen) Verbindungen.

**7.** Verwendung der Verbindungen der allgemeinen Formel (I) nach Anspruch 6 als Zwischenprodukte zur Herstellung von Chinolon-, Naphthyridinon-, Pyridobenzoxazin-, Isothiazolochinolin-, Thiazetochinolon-, Benzochinolizin-, Benzothiazolochinolon-, Pyridobenzothiazin-, Benzoxazolochinolon- und Epoxymethanothiazolochinolon-Derivaten.